# EUROPÄISCHE PATENTSCHRIFT

(11) **EP 1 141 374 B1**
(45) Veröffentlichungstag und Bekanntmachung des Hinweises auf die Patenterteilung: **29.10.2003**
(21) Anmeldenummer: 99965556.6
(22) Anmeldetag: 23.12.1999
(51) Int. Cl.: C12P 41/00, C12P 13/02

(54) **VERFAHREN ZUR HERSTELLUNG VON OPTISCH AKTIVEN 1- AMINO-4- (HYDROXYMETHYL)- CYCLOPENT-2- EN-DERIVATEN**
METHOD FOR PRODUCING OPTICALLY ACTIVE 1-AMINO-4-(HYDROXYMETHYL)-CYCLOPENT-2-ENE DERIVATIVES
PROCEDE DE PRODUCTION DE DERIVES 1-AMINO-4-(HYDROXYMETYL)-CYCLOPENT-2-ENE OPTIQUEMENT ACTIFS

(30) Priorität: 23.12.1998 EP 98124570
(43) Veröffentlichungstag der Anmeldung: 10.10.2001
(73) Patentinhaber: Lonza AG, 4052 Basel (CH)
(72) Erfinder: BRIEDEN, Walter, CH-3902 Brig-Glis (CH); ETTER, Kay-Sara, CH-3945 Niedergampel (CH); PETERSEN, Michael, CH-3930 Visp (CH)
(74) Vertreter: Ritthaler, Wolfgang, Dr.rer.nat.Dipl.-Chem.
(86) Internationale Anmeldenummer: EP9910382
(87) Internationale Veröffentlichungsnummer: WO00039324

(56) Entgegenhaltungen:
- EP-A- 0 878 548
- EP-A- 0 926 131
- WO-A-92/18444
- WO-A-97/45529
- MAHMOUDIAN M ET AL.: "Resolution of 4-amino-cyclopentanecarboxylic acid methyl esters using hydrolytic enzymes" ENZYME AND MICROBIAL TECHNOLOGY, Bd. 14, Nr. 11, November 1992 (1992-11), Seiten 911-916, XP000914640
- JAEGER K-E ET AL.: "Microbial lipases form versatile tools for biotechnology" TRENDS IN BIOTECHNOLOGY, Bd. 16, Nr. 9, September 1998 (1998-09), Seiten 396-403, XP004173187

## Beschreibung

Die vorliegende Erfindung betrifft ein neues Verfahren zur Herstellung von enantiomerenangereicherten 1-Amino-4-(hydroxymethyl)-cyclopent-2-en-Derivaten der allgemeinen Formeln 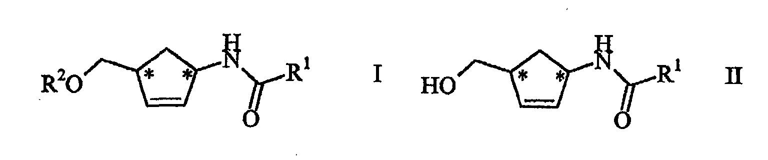 worin R¹ Wasserstoff, Alkyl, Aryl oder Cycloalkyl und R² Acyl bedeutet, sowie insbesondere deren Weiterumsetzung zu den entsprechenden enantiomerenangereicherten 1-Amino-4-(hydroxymethyl)-cyclopent-2-en-Verbindungen der Formel IV.

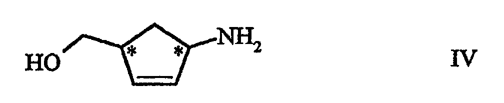

Enantiomerenangereichertes 1-Amino-4-(hydroxymethyl)-cyclopent-2-en der Formel IV, wie z. B. (1R,4S)-1-Amino-4-(hydroxymethyl)-cyclopent-2-en, ist ein wichtiges Zwischenprodukt zur Herstellung von carbocyclischen Nukleosiden wie z. B. Carbovir (Campbell et al., J. Org. Chem. 1995, 60, 4602-4616).

Unter "enantiomerenangereicherten" Verbindungen werden im folgenden Verbindungen mit einem Enantiomeren-Überschuss (ee) von mehr als 20% verstanden.

Bisher sind mehrere Verfahren zur Herstellung von (1R,4S)-1-Amino-4-(hydroxymethyl)-cyclopent-2-en bekannt. Beispielsweise beschreibt die WO 97/45529 ein biotechnologisches Verfahren zur Herstellung von (1R,4S)-1-Amino-4-(hydroxymethyl)-cyclopent-2-en ausgehend von racemischem cis-N-Acetyl-1-amino-4-(hydroxymethyl)-cyclopent-2-en mittels Mikroorganismen, welche letzteres als einzige C-, einzige N- oder als einzige C- und N-Quelle verwerten. Nachteilig bei diesem Verfahren ist, dass es in einem Fermenter durchgeführt werden muss.

In der WO 92/18444 wird eine Hydrolase-katalysierte Acylierung von Cyclopent-2-en-Derivaten ausgehend von racemischem 1-Hydroxy-4-(trityloxymethyl)-cyclopent-2-en beschrieben.

Aufgabe der vorliegenden Erfindung war es, ein alternatives, einfaches und kostengünstiges Verfahren zur Herstellung von enantiomerenangereicherten 1-Amino-4-(hydroxymethyl)-cyclopent-2-en-Derivaten der Formeln I und II und von enantiomerenangereicherten 1-Amino-4-(hydroxymethyl)-cyclopent-2-en-Verbindungen der Formel IV zur Verfügung zu stellen.

Zur Lösung dieser Aufgabe wird erfindungsgemäss entsprechend Anspruch 1 ein racemisches 1-Amino-4-(hydroxymethyl)-cyclopent-2-en-Derivat der allgemeinen Formel 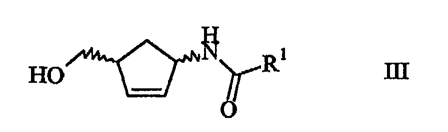 worin R¹ Wasserstoff oder einen gegebenenfalls substituierten C₁₋₈-Alkylrest, linear oder verzweigt, Arylrest oder Cycloalkylrest bedeutet, mittels einer Hydrolase in Gegenwart eines Acylierungsmittels in die enantiomerenangereicherten 1-Amino-4-(hydroxymethyl)-cyclopent-2-en-Derivate der allgemeinen Formeln 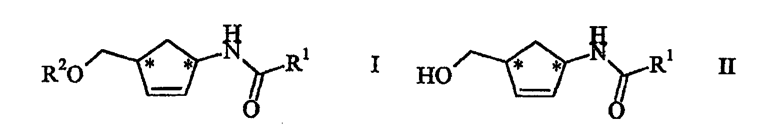 worin R¹ die genannte Bedeutung hat und R² gegebenenfalls substituiertes Acyl bedeutet, überführt.

Gemäß dem Verfahren nach Anspruch 5 wird ein racemisches 1-Amino-4-(hydroxmethyl)-cyclopent-2-en-Derivat der allgemeinen Formel 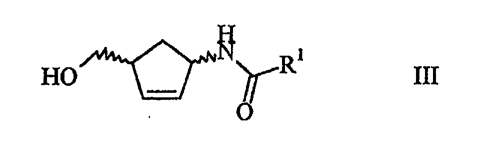 worin R¹ die oben genannte Bedeutung hat, mittels einer Hydrolase in Gegenwart eines Acylierungsmittels in enantiomerenangereicherte 1-Amino-4-(hydroxymethyl)-cyclopent-2en-Derivate der allgemeinen Formel I 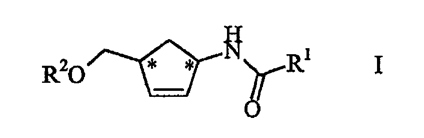 worin R¹ und R² die oben genannte Bedeutung haben, überführt und diese werden dann chemisch zu den entsprechenden Enantiomeren der allgemeinen Formel 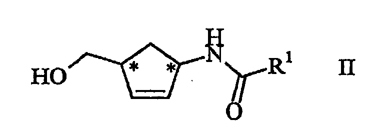 hydrolysiert.

Gemäß dem Verfahren nach Anspruch 7 wird ein racemisches 1-Amino-4-(hydroxymethyl)-cyclopent-2-en-Derivat der allgemeinen Formel 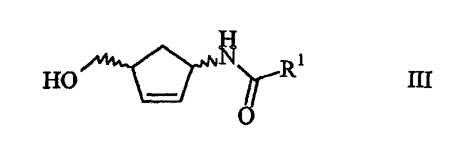 worin R¹ die oben genannte Bedeutung hat, mittels einer Hydrolase in Gegenwart eines Acylierungsmittels in enantiomerenangereicherte 1-Amino-4-(hydroxymethyl)-cyclopent-2-en-Derivate der allgemeinen Formel 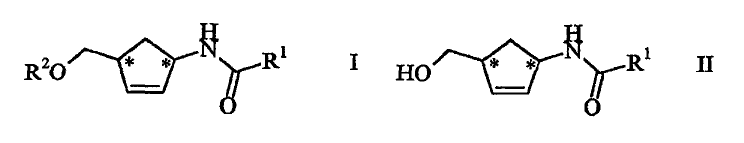 worin R¹ und R² die oben genannte Bedeutung haben, überführt und diese werden dann chemisch zu den entsprechenden enantiomerenangereicherten 1-Amino-4-(hydroxymethyl)-cyclopent-2-en-Isomeren der Formel 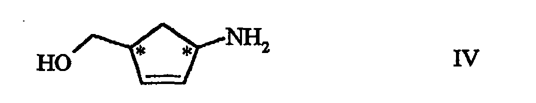 hydrolysiert.

Die Edukte, die racemischen 1-Amino-4-(hydroxymethyl)-cyclopent-2-en -Derivate der allgemeinen Formel III, können ausgehend vom (±)-2-Azabicylco[2.2 1]hept-5-en-3-on gemäss der WO 97/45529 hergestellt werden. Bevorzugt werden die cis-racemischen Edukte eingesetzt

Der Begriff Alkyl, wie er hier verwendet wird, umfaßt sowohl lineares als auch verzweigtes Alkyl Alkyl kann substituiert oder unsubstituiert sein C₁₋₈-Alkyl, steht insbesondere für Methyl, Ethyl, Propyl, Isopropyl, Butyl, tert-Butyl, Pentyl und seine Isomere, Hexyl und seine Isomere, Heptyl und seine Isomere oder Octyl und seine Isomere. Unter substituiertem C₁₋₈-Alkyl ist mit einem oder mehreren Halogenatomen substituiertes C₁₋₈-Alkyl, mit OR³ oder mit NR³R⁴⁻ substituiertes C₁₋₈-Alkyl, worin R³ und R⁴ gleich oder verschieden sind und Wasserstoff oder verzweigtes oder lineares C₁₋₈-Alkyl, Aryl oder Cycloalkyl bedeutet, zu verstehen Als Halogenatom kann F, Cl, Br oder J eingesetzt werden. Als NR³R⁴ ist beispielhaft Methylamino, N-Methyl-N-ethylamino, 1-Piperidinyl oder Aminomethyl zu nennen. Als OR³ sind beispielhaft Methoxy, Methoxymethyl, Ethoxy, Propoxy und Phenoxy zu nennen.

Unter Aryl wird bevorzugt Benzyl oder Phenyl, substituiert oder unsubstituiert, verstanden Unter substituiertem Aryl wird im folgenden mit einem oder mehreren Halogenatomen, C₁₋₄-Alkylgruppen, C₁₋₄-Alkoxygruppen, Amino-, Cyan- oder Nitrogruppen substituiertes Aryl verstanden. Als substituiertes Benzyl wird vorzugsweise Chlor- oder Brombenzyl und als substituiertes Phenyl vorzugsweise Brom- oder Chlorphenyl eingesetzt.

Cycloalkyl ist zweckmäßig C₃₋₇-Cycloalkyl, substituiert oder unsubstituiert, beispielsweise Cyclopropyl, Cyclopentyl oder Cyclohexyl. Geeignete Substituenten sind beispielsweise die für Aryl genannten Substituenten.

Acyl entspricht der Säurekomponente des eingesetzten Acylierungsmittels.

Vorzugsweise bedeutet Acyl C₁₋₆-Alkanoyl, unsubstituiert oder substituiert mit einem oder mehreren Halogenatomen, C₁₋₄-Alkoxy, Aryl, Hydroxy, Amino, Cyan Nitro, und/oder COOR, worin R C₁₋₄-Alkyl bedeutet. Beispiele für unsubstituierte oder substituierte Acylreste sind Acetyl, Propionyl, Butyryl, Chloracetyl, Bromacetyl, Dichloracetyl, Cyanacetyl, Methoxycarbonyl, Ethoxycarbonyl, Methoxyethanoyl, Hydroxybutyroyl, Hydroxyhexanoyl, Phenylcarbonyl, Chlorphenylcarbonyl und Benzylcarbonyl.

Als Acylierungsmittel sind generell Carbonsäurederivate wie Carbonsäureamide, Carbonsäureanhydride oder Carbonsäureester geeignet.

Als Carbonsäureester können Alkoxycarbonsäureester wie Methoxyessigsäureethylester, Methoxyessigsäureisopropylester, C₁₋₆ Carbonsäureester wie Essigsäurebutylester, Buttersäureethylester, Buttersäurephenylester, Buttersäuretrichlorethylester, Hexansäureethylester, Vinylbutyrat, Glycerinester wie Tributyrin (Glycerintributyrat), Glykolester wie Glykoldibutyrat, Diglykolsäurediethylester oder Dicarbonsäureester wie Vinylsuccinat, Cyan-substituierte Ester wie Cyanessigsäureester oder cyclische Carbonsäureester wie Butyrolacton, Caprolacton eingesetzt werden.

Als Carbonsäureamide können die den erwähnten Estern entsprechenden Amide eingesetzt werden.

Als Carbonsäureanhydride können einfache, gemischte oder cyclische Anhydride wie Buttersäureanhydrid, Acetylbenzoat und Bernsteinsäureanhydrid eingesetzt werden.

Als Hydrolasen können Lipasen, Esterasen oder Proteasen eingesetzt werden. Als Lipase können handelsübliche Lipasen verwendet werden wie beispielsweise Novo-Lipase SP523 aus Aspergillus oryzae (Novozym 398), Novo-Lipase SP524 aus Aspergillus oryzae (Lipase = Palatase 20000L von Novo), Novo-Lipase SP525 aus Candida antarctica (Lipase B Novozym 435, immobilisiert), Novo-Lipase SP526 aus Candida antarctica (Lipase A = Novozym 735, immobilisiert), Lipase-kits von Fluka (1 & 2), Amano P Lipase, Lipase aus Pseudomonas sp., Lipase aus Candida cylindracea, Lipase aus Candida lipolytica, Lipase aus Mucor miehei, Lipase M aus Mucor javanicus (Amano), Lipase aus Aspergillus niger, Lipase aus Bacillus thermocatenulatus, Lipase aus Candida antarctica, Lipase AH (Amano; immobilisiert), Lipase P (Nagase), Lipase AY aus Candida rugosa, Lipase G (Amano 50), Lipase F (Amano F-AP15), Lipase PS (Amano), Lipase AH (Amano), Lipase D (Amano), Lipase AK aus Pseudomonas fluorescens, Lipase PS aus Pseudomonas cepacia, Newlase I aus Rhizopus niveus, Lipase PS-CI (immobilisierte Lipase aus Pseudomonas cepacia).

Diese Lipasen können, wie fachmännisch bekannt, als zellfreie Enzymextrakte oder auch in der entsprechenden Mikroorganismenzelle angewendet werden.

Als Proteasen sind ebenfalls handelsübliche geeignet, z. B. Serinproteasen wie Subtilisine. Als Subtilisin können z.B. Savinase aus Bacillus sp., Alcalase, Subtilisin aus Bacillus licheniformis sowie Proteasen aus Aspergillus, Rhizopus, Streptomyces oder Bacillus sp. verwendet werden.

Abhängig von der Wahl der Hydrolase wird in einem racemischen, beispielsweise cis-racemischen, 1-Amino-4-(hydroxymethyl)-cyclopent-2-en der Formel III eines der beiden Enantiomeren acyliert (Verbindungen der Formel I), während das andere Enantiomer unverändert bleibt (Verbindungen der Formel II). Die beiden Enantiomeren können dann getrennt werden.

Unterschiedliche Hydrolasen können unterschiedliche Stereospezifitäten besitzen. Wird beispielsweise cis-N-Acetyl-1-amino-4-(hydroxymethyl)-cyclopent-2-en mit Lipase M und einem Acylierungsmittel umgesetzt, so wird spezifisch das (1R, 4S)-Enantiomer acyliert, das vom nicht-acylierten (1S, 4R)-Enantiomer abgetrennt werden kann. Wird als Hydrolase beispielsweise Savinase (Protease aus Bacillus sp.) verwendet, so wird spezifisch das (1S, 4R)-Enantiomer acyliert, während das (1R, 4S)-Enantiomer zurückbleibt.

Zweckmässig wird die Hydrolase-katalysierte Acylierung bei einer Temperatur von 0 bis 70 °C, vorzugsweise bei einer Temperatur von 15 bis 45 °C durchgeführt.

Die Hydrolase-katalysierte Acylierung kann in einem protischen oder aprotischen organischen Lösungsmittel durchgeführt werden. Als aprotische organische Lösungsmittel sind Ether, aliphatische Kohlenwasserstoffe, organische Basen und Carbonsäurederivate geeignet. Als Ether können tert-Butylmethylether, Diisopropylether, Dibutylether, Dioxan oder Tetrahydrofuran eingesetzt werden. Als aliphatische Kohlenwasserstoffe sind Hexan, Heptan, Octan geeignet. Als organische Basen sind Pyridine oder Trialkylamine wie Triethylamin geeignet. Als Carbonsäurederivate sind bspw. Ethylacetat oder die zuvor beschriebenen Acylierungsmittel möglich.

Die bei der Hydrolase-katalysierten Acylierung gebildeten enantiomerenangereicherten 1-Amino-4-(hydroxymethyl)-cyclopent-2-en-Derivate der allgemeinen Formel I oder II können nach Trennung chemisch direkt zu den entsprechenden enantiomerenangereicherten 1-Amino-4-(hydroxymethyl)-cyclopent-2-en-Isomeren der Formel IV 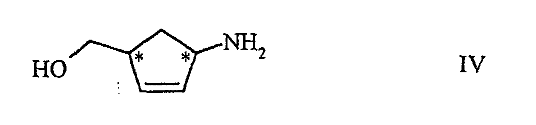 hydrolysiert werden. Alternativ kann das abgetrennte enantiomerenangereicherte 1-Amino-4-(hydroxymethyl)-cyclopent-2-en-Derivats der allgemeinen Formel I durch geeignete Wahl der Hydrolysebedingungen stufenweise zunächst wieder zum entsprechenden enantiomerenangereichterten 1-Amino-4-(hydroxymethyl)-cyclopent-2-en-Derivat der allgemeinen Formel II hydrolysiert werden, welches dann, falls gewünscht, durch weitere chemische Hydrolyse wie oben zum entsprechenden enantiomerenangereicherten 1-Amino-4-(hydroxymethyl)-cyclopent-2-en der Formel IV umgesetzt wird.

Zweckmässig wird die chemische Hydrolyse mit einem Alkalimetallhydroxid oder mit Ammoniak durchgeführt. Als Alkalimetallhydroxid kann Natrium- oder Kaliumhydroxid eingesetzt werden.

Die chemische Hydrolyse kann bei einer Temperatur von 20 bis 100 °C, vorzugsweise bei einer Temperatur von 60 bis 80 °C, durchgeführt werden.

Bevorzugtes enantiomerenangereichertes 1-Amino-4-(hydroxymethyl)-cyclopent-2-en-Derivat der allgemeinen Formel I ist das (1R,4S)- und (1S,4R)-N-Acetyl-1-amino-4-(propylcarbonyloxymethyl)-cyclopent-2-en (R¹ = CH₃, R² = Propylcarbonyl), bevorzugtes 1-Amino-4-(hydroxymethyl)-cyclopent-2-en-Derivate der allgemeinen Formel II ist das (1R,4S)- und (1S,4R)-N-Acetyl-1-amino-4-(hydroxymethyl)-cyclopent-2-en, welche dann chemisch bevorzugt in das (1R,4S)- bzw. (1S,4R)-1-Amino-4-(hydroxymethyl)-cyclopent-2-en hydrolysiert werden.

### Beispiele

### Beispiel 1

50 mg cis-N-Acetyl-1-amino-4-(hydroxymethyl)-cyclopent-2-en und 250 µl Vinylbutyrat wurden in 5 ml 2-Methyl-2-butanol gelöst. 300 mg Lipase M (aus Mucor javanicus; Amano) wurden hinzugegeben und die Suspension bei Raumtemperatur gerührt. Nach 16 h lag (1S, 4R)-N-Acetyl-1-amino-4-(hydroxymethyl)-cyclopent-2-en mit einem Enantiomerenüberschuss von 98,5% (GC) vor.

Nach Trennung von (1S, 4R)-N-Acetyl-1-amino-4-(hydroxymethyl)-cyclopent-2-en und gebildetem (1R, 4S)-N-Acetyl-1-amino-4-(propylcarbonyloxymethyl)-cyclopent-2-en (Chromatographie an Kieselgel 60) wurden beide Verbindungen getrennt in 2M Natronlauge aufgenommen. (1S, 4R)-N-Acetyl-1-amino-4-(hydroxymethyl)-cyclopent-2-en wurde durch Rühren bei 80 °C (70 h) in das enantiomerenreine bzw. -angereicherte cis-1-Amino-4-(hydroxymethyl)-cyclopent-2-en überführt, während (1R, 4S)-N-Acetyl-1-amino-4-(propylcarbonyloxymethyl)-cyclopent-2-en durch Rühren bei Raumtemperatur (5 h) in das enantiomerenreine bzw. -angereichterte cis-N-Acetyl-1-amino-4-(hydroxymethyl)-cyclopent-2-en überführt wurde.

### Beispiel 2

10 mg cis-N-Acetyl-1-amino-4-(hydroxymethyl)-cyclopent-2-en und 50 µl Vinylbutyrat wurden in 1 ml Dioxan gelöst. 30 mg Lipase M (aus Mucor javanicus; Amano) wurden hinzugegeben und die Suspension bei Raumtemperatur gerührt. Nach 20 h lag (1S, 4R)-N-Acetyl-1-amino-4-(hydroxymethyl)-cyclopent-2-en mit einem Enantiomerenüberschuss von 91,0% (GC) vor.

### Beispiel 3

10 mg cis-N-Acetyl-1-amino-4-(hydroxymethyl)-cyclopent-2-en und 50 µl Vinylbutyrat wurden in 1 ml 2-Methyl-2-butanol gelöst. 40 mg Savinase (Protease aus Bacillus sp.; Novo Nordisk) wurden hinzugegeben und die Suspension bei Raumtemperatur gerührt. Nach 20 h lag (1R, 4S)-N-Acetyl-1-amino-4-(hydroxymethyl)-cyclopent-2-en mit einem Enantiomerenüberschuss von 91,7% (GC) vor.

### Beispiel 4

10 mg cis-N-Acetyl-1-amino-4-(hydroxymethyl)-cyclopent-2-en und 50 µl Vinylbutyrat wurden in 1 ml Dioxan gelöst. 40 mg Savinase (Protease aus Bacillus sp.; Novo Nordisk) wurden hinzugegeben und die Suspension bei Raumtemperatur gerührt. Nach 200 h lag (1R, 4S)-N-Acetyl-1-amino-4-(hydroxymethyl)-cyclopent-2-en mit einem Enantiomerenüberschuss von 81,7% (GC) vor.

### Beispiel 5

100 mg cis-N-Acetyl-1-amino-4-(hydroxymethyl)-cyclopent-2-en und 0.5 mmol Vinylbutyrat wurden in 1 ml 2-Methyl-2-butanol gelöst. 20 mg Lipase PS (aus Pseudomonas cepacia) wurden hinzugegeben und die Suspension bei Raumtemperatur gerührt. Nach 21 h liegt (1R, 4S)-N-Acetyl-1-amino-4-(hydroxymethyl)-cyclopent-2-en mit einem Enantiomerenüberschuss von 44% (GC) vor.

### Beispiel 6

10 mg cis-N-Acetyl-1-amino-4-(hydroxymethyl)-cyclopent-2-en und 0.03 mmol Tributyrin wurden in 1 ml 2-Methyl-2-butanol gelöst. 20 mg Lipase PS (Pseudomonas cepacia) wurden hinzugegeben und die Suspension bei Raumtemperatur gerührt. Nach 200 h liegt (1R, 4S)-N-Acetyl-1-amino-4-(hydroxymethyl)-cyclopent-2-en mit einem Enantiomerenüberschuss von 32% (GC) vor.

## Patentansprüche

1. Verfahren zur Herstellung von enantiomerenangereicherten 1-Amino-4-(hydroxymethyl)-cyclopent-2-en-Derivaten der allgemeinen Formeln 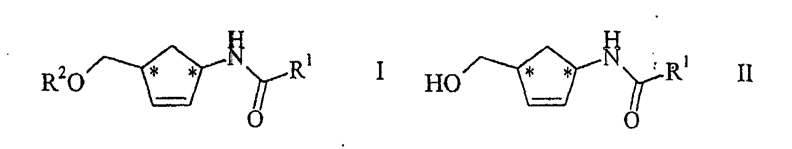 worin R¹ Wasserstoff oder einen gegebenenfalls substituierten C₁₋₈-Alkylrest, Arylrest oder Cycloalkylrest bedeutet und R² gegebenenfalls substituiertes Acyl, bedeutet, umfassend die Umsetzung eines racemischen 1-Amino-4-(hydroxmethyl)-cyclopent-2-en-Derivats der allgemeinen Formel 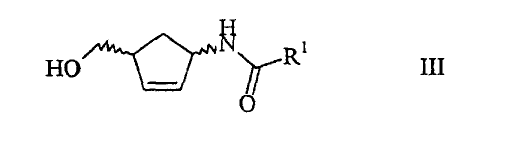 worin R¹ die genannte Bedeutung hat, mittels einer Hydrolase in Gegenwart eines Acylierungsmittels.

2. Verfahren nach Anspruch 1, **dadurch gekennzeichnet, dass** als Hydrolase eine Protease, Esterase oder Lipase einsetzt wird.

3. Verfahren nach Anspruch 1 oder 2, **dadurch gekennzeichnet, dass** die Hydrolase-katalysierte Acylierung bei einer Temperatur von 0 bis 70 °C durchgeführt wird.

4. Verfahren nach einem der Ansprüche 1 bis 3, **dadurch gekennzeichnet, dass** die Hydrolase-katalysierte Acylierung in einem protischen oder aprotischen organischen Lösungsmittel durchgeführt wird.

5. Verfahren zur Herstellung von enantiomerenangereicherten 1-Amino-4-(hydroxymethyl)-cyclopent-2-en-Derivaten der allgemeinen Formel 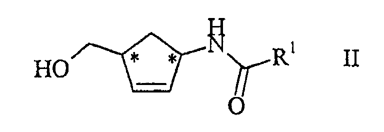 **dadurch gekennzeichnet, dass** man ein racemisches 1-Amino-4-(hydroxmethyl)-cyclopent-2-en-Derivat der allgemeinen Formel 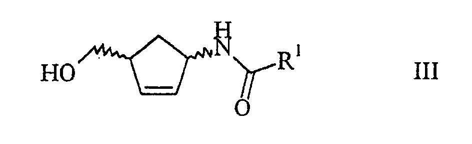 worin R¹ die in Anspruch 1 genannte Bedeutung hat, mittels einer Hydrolase in Gegenwart eines Acylierungsmittels in enantiomerenangereicherte 1-Amino-4-(hydroxymethyl)-cyclopent-2en-Derivate der allgemeinen Formel I 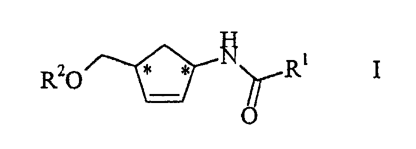 worin R¹ und R² die in Anspruch 1 genannte Bedeutung haben, überführt und diese dann chemisch zu den entsprechenden Enantiomeren der allgemeinen Formel II hydrolysiert.

6. Verfahren nach Anspruch 5, **dadurch gekennzeichnet, dass** die chemische Hydrolyse bei einer Temperatur von 20 bis 100 °C durchgeführt wird.

7. Verfahren zur Herstellung von enantiomerenangereichertem 1-Amino-4-(hydroxymethyl)-cyclopent-2-en der Formel 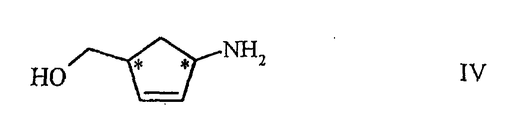 **dadurch gekennzeichnet, dass** man ein racemisches 1-Amino-4-(hydroxymethyl)-cyclopent-2-en-Derivat der allgemeinen Formel 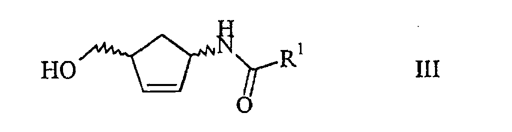 worin R¹ die in Anspruch 1 genannte Bedeutung hat, mittels einer Hydrolase in Gegenwart eines Acylierungsmittels in enantiomerenangereicherte 1-Amino-4-(hydroxymethyl)-cyclopent-2-en-Derivate der allgemeinen Formel 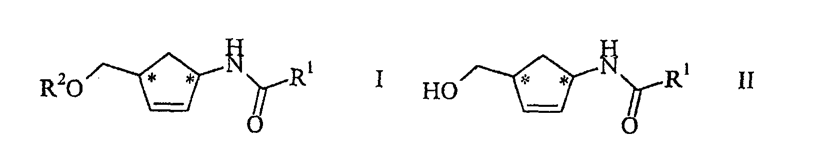 worin R¹ und R² die in Anspruch 1 genannte Bedeutung haben, überführt und diese dann chemisch zu den entsprechenden enantiomerenangereicherten 1-Amino-4-(hydroxymethyl)-cyclopent-2-en-Isomeren der Formel IV hydrolysiert.

## Claims

1. Process for preparing enantiomerically enriched 1-amino-4-(hydroxymethyl)cyclopent-2-ene derivatives of the general formulae 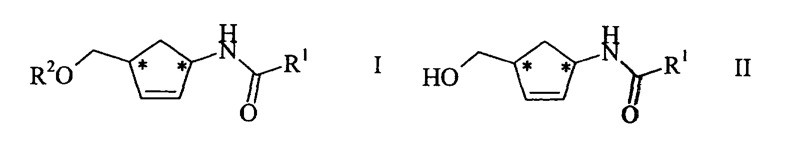 in which R¹ is hydrogen or an optionally substituted C₁₋₈-alkyl radical, aryl radical or cycloalkyl radical and R² is an optionally substituted acyl, in that a racemic 1-amino-4-(hydroxymethyl)cyclopent-2-ene derivative of the general formula 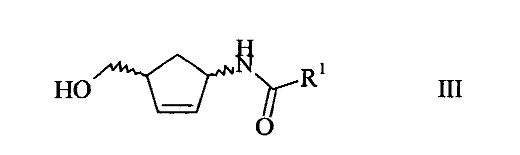 in which R¹ is as defined above is reacted using a hydrolase in the presence of an acylating agent.

2. Process according to Claim 1, **characterized in that** the hydrolase used is a protease, esterase or lipase.

3. Process according to Claim 1 or 2, **characterized in that** the hydrolase-catalyzed acylation is carried out at a temperature of from 0 to 70°C.

4. Process according to one of Claims 1 to 3, **characterized in that** the hydrolase-catalyzed acylation is carried out in a protic or aprotic organic solvent.

5. Process for preparing enantiomerically enriched 1-amino-4-(hydroxymethyl)cyclopent-2-ene derivatives of the general formula 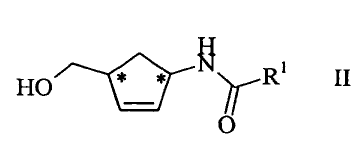 **characterized in that** a racemic 1-amino-4-(hydroxymethylycyclopent-2-ene derivative of the general formula 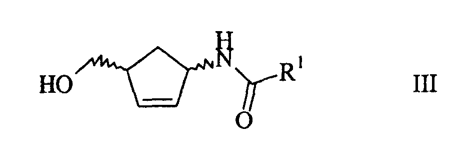 in which R¹ is as defined in Claim 1 is converted using a hydrolase in the presence of an acylating agent into enantiomerically enriched 1-amino-4-(hydroxymethyl)cyclopent-2-ene derivatives of the general formula I 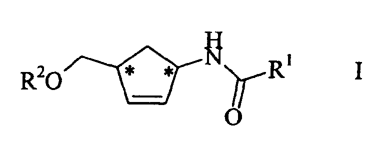 in which R¹ and R² are as defined in Claim 1, and these are then chemically hydrolyzed into the corresponding enantiomers of the general formula II.

6. Process according to Claim 5, **characterized in that** the chemical hydrolysis is carried out at a temperature of from 20 to 100°C.

7. Process for preparing enantiomerically enriched 1-amino-4-(hydroxymethyl)cyclopent-2-ene of the formula 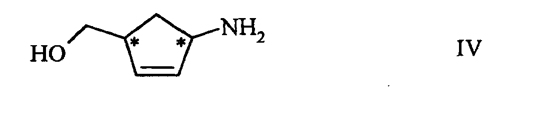 **characterized in that** a racemic 1-amino-4-(hydroxymethyl)cyclopent-2-ene derivative of the general formula 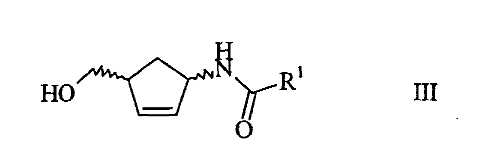 in which R¹ is as defined in Claim 1 is converted using a hydrolase in the presence of an acylating agent into enantiomerically enriched 1-amino-4-(hydroxymethyl)cyclopent-2-ene derivatives of the general formula 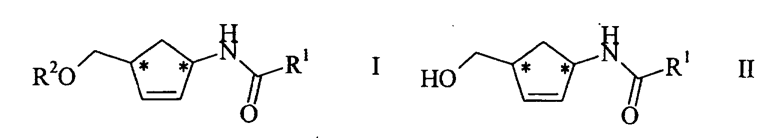 in which R¹ and R² are as defined in Claim 1, and these are then chemically hydrolyzed into the corresponding enantiomerically enriched 1-amino-4-(hydroxymethyl)cyclopent-2-ene

## Revendications

1. Procédé en vue de la préparation de dérivés 1-amino-4-(hydroxyméthyl)-cyclopent-2-èniques, enrichis en énantiomères, de la formule générale 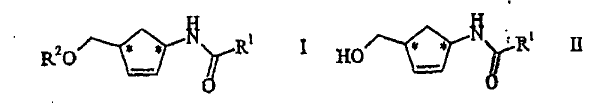 dans lesquelles R¹ représente l'hydrogène ou un résidu C₁₋₈-alkyle, le cas échéant, substitué, un résidu aryle ou un résidu cycloalkyle et R² représente un résidu acyle, le cas échéant, substitué, comprenant la réaction d'un dérivé racémique du 1-amino-4-(hydroxyméthyl)-cyclopent-2-ène de la formule générale 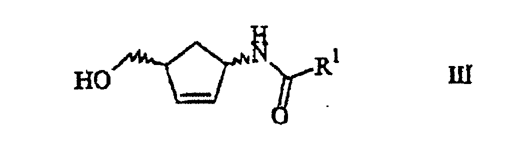 dans laquelle R¹ a la signification citée, par l'intermédiaire d'une hydrolase en présence d'un agent d'acylation.

2. Procédé selon la revendication 1, **caractérisé en ce que** l'on utilise, en tant qu'hydrolase, une protéase, une estérase ou une lipase.

3. Procédé selon la revendication 1 ou la revendication 2, **caractérisé en ce que** l'acylation catalysée par des hydrolases est effectuée à une température de 0 à 70°C.

4. Procédé selon l'une quelconque des revendications 1 à 3, **caractérisé en ce que** l'acylation catalysée par des hydrolases est effectuée dans un solvant organique protique ou aprotique.

5. Procédé en vue de la préparation de dérivés 1-amino-4-(hydroxyméthyl)-cyclopent-2-èniques, enrichis en énantionmères, de la formule générale 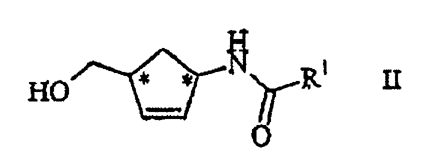 **caractérisé en ce que** l'on convertit un dérivé racémique du 1-amino-4-(hydroxyméthyl)-cyclopent-2-ène de la formule générale 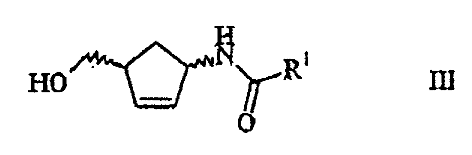 où R¹ a la signification citée dans la revendication 1, à l'aide d'une hydrolase en présence d'un agent d'acylation en dérivés enrichis en énantiomères, 1-amino-4-(hydroxyméthyl)-cyclopent-2-èniques de la formule générale 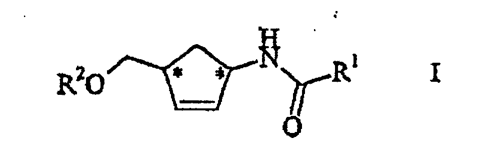 où R¹ et R² ont la signification citée dans la revendication 1, et **en ce que** l'on hydrolyse alors ceux-ci par voie chimique pour former les énantiomères correspondants de la formule générale II.

6. Procédé selon la revendication 5, **caractérisé en ce que** l'hydrolyse chimique est effectuée à une température de 20 à 100°C.

7. Procédé en vue de la préparation de 1-amino-4-(hydroxyméthyl)-cyclopent-2-ène, enrichi en énantiomères, de la formule 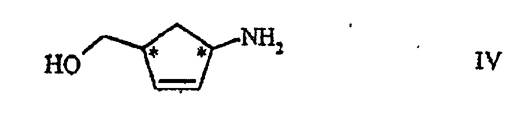 **caractérisé en ce que** l'on convertit un dérivé racémique du 1-amino-4-(hydroxyméthyl)-cyclopent-2-ène de la formule générale 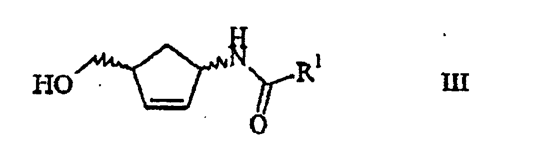 où R¹ a la signification citée dans la revendication 1, à l'aide d'une hydrolase en présence d'un agent d'acylation, en des dérivés, enrichis en énantiomères, 1-amino-4-(hydroxyméthyl)-cyclopent-2-èniques de la formule générale 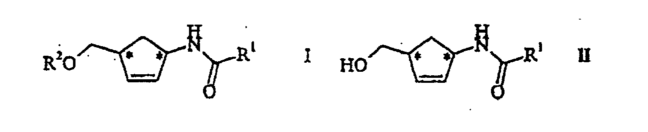 où R¹ et R² ont la signification citée dans la revendication 1, et **en ce que** l'on hydrolyse alors ceux-ci par voie chimique pour former les isomères correspondants, enrichis en énantiomères, 1-amino-4-(hydroxyméthyl)-cyclopent-2-èniques de la formule IV.
